# EUROPEAN PATENT APPLICATION

(11) **EP 4 420 718 A1**
(43) Date of publication of application: **28.08.2024**
(21) Application number: 24156400.4
(22) Date of filing: 07.02.2024
(51) Int. Cl.: A61N 5/06

(54) **DEVICE FOR MEASURING SURFACES TO BE TREATED BY PHOTOBIOMODULATION AND PHOTOBIOMODULATION APPARATUS WITH SUCH A DEVICE**

(30) Priority: 27.02.2023 IT 202300003393
(71) Applicant: ASA S.r.l., 36057 Arcugnano (VI) (IT)
(72) Inventor: MARCHESINI, Roberto, 36015 SCHIO VI (IT); VITULO, Silvia, 36100 VICENZA (IT)
(74) Representative: Modiano, Micaela Nadia

(57) **Abstract**

A device (10) for measuring surfaces (12) to be treated by photobiomodulation, comprising:
- optical means (14) for tracking axes (X, Y) of a surface (12) to be treated,
- data communication means for communicating data (15) to a data processing unit (16), the data communication means (15) being connected to the optical means (14),
- a body (17) for the containment of the optical means (14) and of the data communication means (15), the body (17) being ergonomic.

## Description

The present invention relates to a device for measuring surfaces to be treated by photobiomodulation.

The invention also relates to a photobiomodulation apparatus with such a device.

For several decades, photobiomodulation has been used successfully in the treatment of various musculoskeletal disorders and in tissue repair.

Photobiomodulation therapy (PBMT) is a physical therapy that uses light emitted by a laser (or other light source) to interact with human and/or animal biological tissues, in order to promote the restoration of tissue homeostasis, reduce inflammation and pain, and facilitate healing processes.

The scientific literature related to PBMT shows that the indication of effective dosage in clinical treatments is generally accepted as "J/cm²," i.e., as energy delivered (in Joules) per unit area treated (in cm²).

The same literature and the scientific community agree in defining that there is no single correct dose value but rather an effective range of dosages for a given disorder and that a minimum dosage is necessary to achieve significant therapeutic results, while an excessive dosage may be counterproductive and/or harmful.

Thus, the need to have energy dosage control is felt in order to ensure high levels of effectiveness, safety, adaptability of the therapy on each patient, and replicability of the treatment.

The correct definition of the therapeutic protocol to be administered to the patient therefore implies knowing not only the energy delivered, which nowadays all PBMT apparatuses are able to report, but also the treatment area onto which the therapeutic light is to be conveyed.

In static "spot-by-spot" treatment, where the treatment area coincides with the area of the spot of the beam of light on the skin, calculation of the effective energy dosage can be easily implemented.

However, dosage control is reduced with light beam application by scanning, since although in some devices it is possible to manually set the parameter of the area to be treated, in practice it is very difficult for the operator to estimate the actual treatment area.

This difficulty is increased if it is necessary to treat different anatomical areas with irregular surfaces in a three-dimensional space.

Generally, measurement instruments are not provided together with PBMT apparatuses, delegating any area estimation activity to the operator performing the therapy.

Usually, therefore, therapy is performed in a crude and uncontrolled manner by delivering a certain amount of energy for a certain application time over an indefinite area, which is not measured in advance.

In some limited cases, analog measuring instruments are used, such as for example tape measures, rulers, or predefined templates, but these have many drawbacks.

Indeed, use of the tape measure and ruler requires area calculations by the operator and/or manual entry of the measured value in the user interface of the apparatus.

The use of templates instead has the drawback of constraining the application to predefined areas, without being able to adapt to the actual physical needs/characteristics of the patient.

Moreover, all solutions of the known type are unsuitable to handle the application on a sequence of areas of different sizes.

The aim of the present invention is to provide a device for measuring surfaces to be treated by photobiomodulation and a photobiomodulation apparatus with such a device that are capable of improving the background art in one or more of the above aspects.

Within this aim, an object of the invention is to provide a device for measuring surfaces to be treated by photobiomodulation and a photobiomodulation apparatus with such a device that allow to trace the treatment region directly on the patient.

Another object of the invention is to provide a device for measuring surfaces to be treated by photobiomodulation and a photobiomodulation apparatus with such a device that allow to correctly and automatically estimate the areas of flat and/or curved anatomical surfaces such as the back, shoulder, ankle, etc., without requiring calculations by the operator.

A further object of the invention is to provide a device for measuring surfaces to be treated by photobiomodulation that can interface with a PBMT apparatus in order to automatically adapt the treatment parameters and be able to guide the operator step by step in the correct distribution of the energy dosage intended for each area.

Another object of the invention is to provide a device for measuring surfaces to be treated by photobiomodulation and a photobiomodulation apparatus with such a device that allow to optimize treatment times with a solution that can be executed rapidly.

A still further object of the present invention is to overcome the drawbacks of the background art in a manner that is alternative to any existing solutions.

Not least object of the invention is to provide a device for measuring surfaces to be treated by photobiomodulation and a photobiomodulation apparatus with such a device that are highly reliable, relatively easy to provide, and at competitive costs.

This aim, as well as these and other objects that will become better apparent hereinafter, are achieved by a device for measuring surfaces to be treated by photobiomodulation, characterized in that it comprises:
- optical means for tracking the axes of a surface to be treated,
- data communication means for communicating data to a data processing unit, said data communication means being connected to said optical means,
- a body for the containment of said optical means and said data communication means, said body being ergonomic.

The above aim and objects and others that will become better apparent hereinafter, are achieved by a photobiomodulation apparatus with such a device.

Further characteristics and advantages of the invention will become better apparent from the description of a preferred but not exclusive embodiment of a device for measuring surfaces to be treated by photobiomodulation and of a photobiomodulation apparatus, according to the invention, illustrated by way of non-limiting example in the accompanying drawings, wherein:
Figure 1 is a view of a device for measuring surfaces to be treated by photobiomodulation and of a photobiomodulation apparatus with such a device, according to the invention, in a first step of use;
Figure 2 is a view of a photobiomodulation apparatus, according to the invention, in a second step of use.

With reference to the figures, a device for measuring surfaces to be treated by photobiomodulation, according to the invention, is generally designated by the reference numeral 10.

The device 10 is associated with a photobiomodulation apparatus 11 and is used for measuring one or more surfaces 12 of a patient 13 to be treated.

The device 10 comprises:
- optical means 14 for tracking the axes X, Y of a surface 12 to be treated,
- data communication means 15 for communicating data to a data processing unit 16, said data communication means 15 being connected to the optical means 14,
- a body 17 for the containment of the optical means 14 and of the data communication means 15.

Advantageously, the body 17 is ergonomic.

In the present description, the term "ergonomic" means that the body 17 can be gripped and handled easily.

The body 17 has a tip 21, at which the optical means 14 are arranged.

Preferably, the device 10 is a mouse pen.

The device 10 is connected to the data processing unit 16 by means of wire and/or wireless means of communication, such as for example a wireless emitter/antenna and a wireless USB plug with wireless receiver, not shown in the figures, which is associated with the apparatus 11.

In particular, the optical means 14 are constituted by an optical sensor.

The data communication means 15 are constituted by a wireless emitter/antenna.

The apparatus 11 comprises:
- a user interface 18, constituted for example by a touchscreen,
- the data processing unit 16, comprising a microprocessor containing software for managing the apparatus 11 and the data received from the device 10, said microprocessor being connected to a receiver of data that arrive from the device 10, not shown in the figures,
- the data receiver, such as for example the wireless USB plug mentioned above,
- a light source 20, adapted to emit therapeutic light,
- a handpiece 19 for directing and delivering the therapeutic light emitted by the light source 20 onto the surface 12 to be treated,
- the device 10, as described above.

It should be noted that the presence of the device 10 separate from handpiece 19 allows the former to be interfaced with different photobiomodulation apparatuses as needed.

Moreover, the device 10 connected by virtue of wireless means to the apparatus 11 allows it to be easy to handle, lightweight, and not constrained by the presence/size of cables and/or fiber optics.

Moreover, such a device 10 is easily removable/replaceable in case of malfunctions and/or breakage.

In other embodiments, not shown in the figures, the device 10 can be integrated in a terminal associated with the handpiece 19 and/or integrated in the handpiece 19.

The use of the device 10 and of the apparatus 11, according to the invention, is as follows.

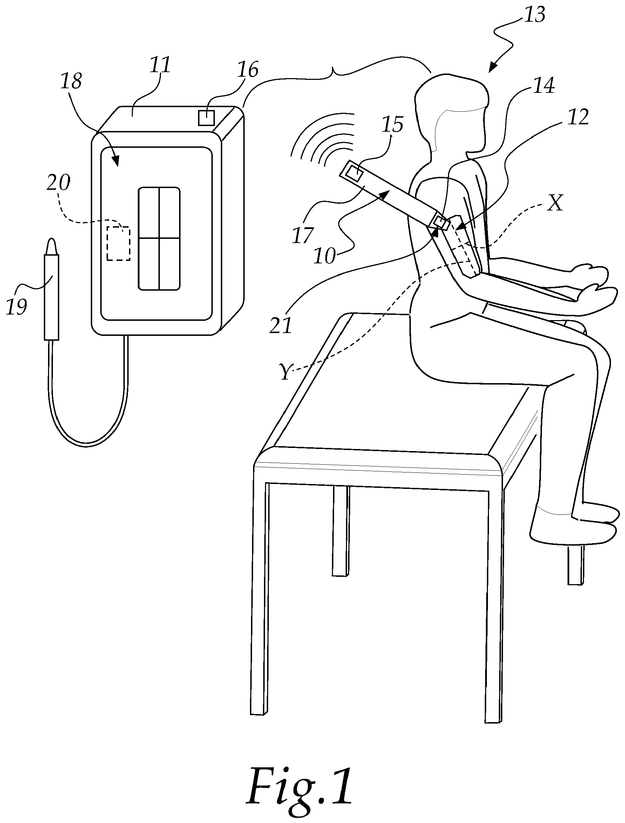

In order to be able to measure a surface 12 of a patient 13 to be treated, the operator tracks the axes X, Y of the surface 12 with the means 14 of the device 10, as shown in Figure 1.

Advantageously, the axes X and Y of the surface 12 are the median axes.

However, the axes X and Y of the surface 12 can also be the segments that correspond to the sides of the perimeter of the surface 12.

The data acquired by the device 10 are sent to the data processing unit 16, which, by means of the software, converts the motion coordinates detected by the optical means 14 into length measurements and calculates the corresponding area.

In practice, the device 10 analyzes in real time the photographs acquired by the optical means 14, comparing them with the previous ones, and the resulting difference matrix is used to calculate the motion vector.

Two values of relative displacement with respect to the two axes X and Y are therefore obtained at each instant.

The device 10 then sends a data packet containing the progressive sequence of relative displacement values to the data processing unit 16.

Simultaneously, the area is represented graphically on the screen of the user interface 18, for example at 1:1 scale.

If multiple surfaces 12 need to be treated, it is possible, by means of the user interface 18, to select the option of adding a new surface to repeat the previous operations, and in this case the software adds the values of the measurements of the lines and/or areas to the previous ones.

By means of the user interface 18 it is also possible to select a further measurement option, without activating the previously described addition function, to record multiple surfaces to be treated but separately.

In this case, the treatment time is divided into a number of intervals equal to the number of lines/surfaces tracked separately and is proportional to the area value associated with each surface (or line) to guide the operator toward the correct distribution of the energy dosage on each area (or line).

If it is necessary to treat lines instead of surfaces, for example the path of nerves, the device 10 operates in a manner that is similar to the case of the surface 12, but tracking of a single line is sufficient.

Once tracking of the surfaces (or lines) to be treated has been performed, the software automatically sets the energy and time values needed to cover the tracked surfaces (or lines) with the predefined or user-set energy dosage.

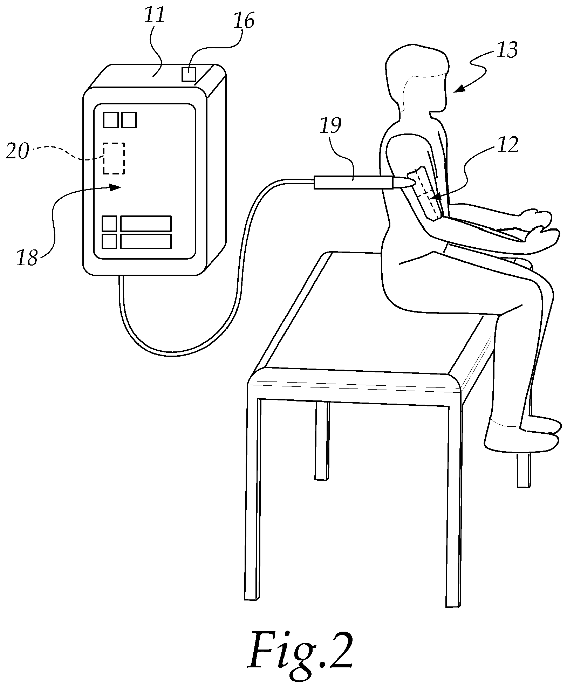

The photobiomodulation treatment is then performed on the patient 13 by means of the handpiece 19, as shown in Figure 2.

It should be noted that the tracking of the axes X, Y of a surface allows, with good approximation, to convert a surface of three-dimensional space, for example a cylindrical or hemispherical surface (such as limbs, knee, shoulder), into a flat surface, for example a rectangular or oval surface, of equal area.

It should also be noted that tracking the axes X, Y of a surface allows to easily maintain the orientation of the device 10 that incorporates the optical sensor 14, allowing a very reliable length measurement, which is not feasible by tracking perimeters or entire surfaces in three-dimensional space, which would inevitably entail unintended rotations of the tracking device, at the same time affecting the correct interpretation of the motion coordinates and thus of the resulting area.

It should be noted that the use of a device and apparatus for photobiomodulation according to the invention allow the operator to have from the outset an estimate of the application times on the basis of the selected parameters and to manage the treatment session optimally, avoiding energy under/overdosage.

Moreover, with a photobiomodulation device and apparatus according to the invention it is possible to perform a therapy that is more targeted and modeled on the actual needs and physical characteristics of the patient than is possible with similar equipment of the known type.

In practice it has been found that the invention achieves the intended aim and objects, providing a device for measuring surfaces to be treated by photobiomodulation and a photobiomodulation apparatus with such a device that allow to track the treatment region directly on the patient.

The invention provides a device for measuring surfaces to be treated by photobiomodulation and a photobiomodulation apparatus with such a device that allow to correctly, and automatically estimate the areas of flat and/or curved anatomical surfaces such as the back, shoulder, ankle, etc., without requiring calculations by the operator.

Moreover, the invention provides a device for measuring surfaces to be treated by photobiomodulation that can interface with a PBMT apparatus in order to automatically adapt the treatment parameters and be able to guide the operator step by step in the correct distribution of the energy dosage intended for each area.

Furthermore, the invention provides a device for measuring surfaces to be treated by photobiomodulation and a photobiomodulation apparatus with such a device that allow to optimize treatment times with a solution that can be executed quickly.

The invention thus conceived is susceptible of numerous modifications and variations, all of which are within the scope of the appended claims; all the details may furthermore be replaced with other technically equivalent elements.

In practice, the materials used, so long as they are compatible with the specific use, as well as the contingent dimensions and shapes, may be any according to the requirements and the state of the art.

The disclosures in Italian Patent Application No. 102023000003393 from which this application claims priority are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device (10) for measuring surfaces (12) to be treated by photobiomodulation, **characterized in that** it comprises:
- optical means (14) for tracking the axes (X, Y) of a surface (12) to be treated,
- data communication means for communicating data (15) to a data processing unit (16), said data communication means (15) being connected to said optical means (14),
- a body (17) for the containment of said optical means (14) and said data communication means (15), said body (17) being ergonomic.

2. The device (10) according to claim 1, **characterized in that** said body (17) is provided with a tip (21), at which said optical means (14) are located.

3. The device (10) according to claim 1, **characterized in that** it is a mouse pen.

4. The device (10) according to claim 1, **characterized in that** said optical means (14) are constituted by an optical sensor.

5. The device (10) according to claim 1, **characterized in that** said data communication means (15) are constituted by a wireless emitter/antenna.

6. A photobiomodulation apparatus (11), **characterized in that** it comprises a device (10) according to one or more of the preceding claims.

7. The apparatus (11) according to claim 6, **characterized in that** it comprises:
- a user interface (18),
- said data processing unit (16) comprising a microprocessor containing software for managing said apparatus (11) and data received from said device (10), said microprocessor being connected to a receiver of data originating from said device (10),
- said data receiver,
- a light source (20) adapted to emit therapeutic light,
- a handpiece (19) for directing and delivering said therapeutic light emitted by said light source (20).

8. The apparatus (11) according to claim 6 or 7, **characterized in that** said user interface (18) is a touchscreen.

9. The apparatus (11) according to one or more of claims 6 to 8, **characterized in that** said data receiver is a wireless USB plug.

10. The apparatus (11) according to one or more of claims 6 to 9, **characterized in that** said device (10) is integrated in a terminal associated with said handpiece (19).

11. The apparatus (11) according to one or more of claims 6 to 9, **characterized in that** said device (10) is integrated in said handpiece (19).
